# EUROPEAN PATENT APPLICATION

(11) **EP 4 806 986 A1**
(43) Date of publication of application: **16.09.2026**
(21) Application number: 24888696.2
(22) Date of filing: 05.11.2024
(51) Int. Cl.: C12N 5/071, C12N 1/00, C12N 5/10

(54) **METHOD FOR PRODUCING CORNEAL ENDOTHELIAL CELLS**

(30) Priority: 06.11.2023 JP 2023189426
(71) Applicant: The University of Osaka, Suita-shi, Osaka 565-0871 (JP)
(72) Inventor: NISHIDA Kohji, Suita-shi, Osaka 565-0871 (JP); BABA Koichi, Suita-shi, Osaka 565-0871 (JP); HARA Susumu, Suita-shi, Osaka 565-0871 (JP)
(74) Representative: Vossius & Partner Patentanwälte Rechtsanwälte mbB
(86) International application number: PCT/JP2024/039276
(87) International publication number: WO 2025/100401

(57) **Abstract**

The present invention provides a method for producing corneal endothelial cells, comprising culturing periocular neural crest cells using a medium containing a fibroblast growth factor. The present invention is able to provide corneal endothelial cells that have characteristics more closely resembling those of corneal endothelial cells in a living body and are of sufficient quality to allow the cells to be transplanted into humans.

## Description

### TECHNICAL FIELD

The present invention relates to a method for producing corneal endothelial cells.

### BACKGROUND ART

The cornea is located at the front of the eyeball and is a transparent tissue with five distinct layers. The corneal endothelial cell layer is a single cell layer that resides in the deeper part of the cornea. The corneal endothelial cell layer has barrier and pump functions and serves to provide constant moisture to the cornea to maintain the transparency of the cornea.

Corneal endothelial diseases, including bullous keratopathy, are serious diseases leading to blindness. These diseases may require corneal transplantation, which requires the donation of the cornea from a donor patient. However, thousands of patients are placed on the waiting list for corneal transplantation per year due to the persistent lack of a sufficient number of donors. There are also other problems, including rejection reaction following transplantation of the cornea from another person. As alternatives, a transplantation method using a corneal epithelium sheet made from stem cells and other methods are currently under development, but the development of a novel therapy for treating corneal endothelial cells is still insufficient.

Induction of human corneal endothelial cells (CECs) from human pluripotent stem cells requires the induction of periocular neural crest followed by the induction of corneal endothelial cells from the periocular neural crest. Although several methods have been proposed, these methods have been limited to generating CEC-like cells (Non-Patent Literature 1). No induction methods for human corneal endothelial cells have yet been developed that are scientifically and clinically verified as sufficient.

The present inventors developed a 2D culture system that reproduces the development of the whole eye from human pluripotent stem cells (Non-Patent Literatures 2 and 3). In this culture system, concentric zonal structures (self-formed ectodermal autonomous multi-zone: SEAM) are induced. The major cell groups that constitute the developing eye (including the corneal epithelium, retina, and lens epithelium) appear at specific sites in the SEAM. Currently, a first-in-human clinical study of a corneal epithelial cell sheet prepared by the SEAM method is ongoing.

The present inventors have reported that the transcription factor TFAP2B (Transcription Factor AP-2 Beta; AP-2β) is important for the differentiation of the neural crest into corneal endothelial cells and the maintenance of the high proliferative ability of corneal endothelial cells (Non-Patent Literature 4).

### CITATION LIST

### NON-PATENT LITERATURE

Non-Patent Literature 1: Jiagang J. Zhao and Natalie A. Afshari, Invest Ophthalmol Vis Sci. 2016 Dec; 57(15): 6878-6884.
Non-Patent Literature 2: Hayashi et al. Nature. 2016 Mar 17;531(7594):376-380.
Non-Patent Literature 3: Hayashi et al. Nature Protocols, 2017, 12(4), 683-696.
Non-Patent Literature 4: Hara et al. J Biol Chem. 2019, 294(7), 2460-2469.

### SUMMARY OF INVENTION

### TECHNICAL PROBLEM

An object of the present invention is to provide a method for producing corneal endothelial cells having characteristics more closely resembling those of corneal endothelial cells in a living body as compared with corneal endothelial cell-like cells produced by a conventional method.

### SOLUTION TO PROBLEM

The present invention was made to solve the above problems and includes the following.
[1] A method for producing corneal endothelial cells, comprising culturing periocular neural crest cells using a medium containing a fibroblast growth factor.
[2] The production method according to the above [1], wherein the medium further contains a glycolytic inhibitor.
[3] The production method according to the above [2], wherein the glycolytic inhibitor is 2-deoxy-D-glucose.
[4] The production method according to any one of the above [1] to [3], wherein the periocular neural crest cells are periocular neural crest cells induced to differentiate from pluripotent stem cells.
[5] The production method according to any one of the above [1] to [3], wherein the periocular neural crest cells are periocular neural crest cells induced to differentiate from pluripotent stem cells via a SEAM cell population.
[6] The production method according to the above [4] or [5], wherein the pluripotent stem cells are human iPS cells.
[7] A method for producing periocular neural crest cells, comprising forming a SEAM cell population from pluripotent stem cells, and culturing cells that form the SEAM cell population using a medium containing a fibroblast growth factor, wherein the cells contain neural crest cells.
[8] The production method according to the above [7], wherein the culturing cells that form the SEAM cell population using a medium containing a fibroblast growth factor comprises dispersing the cells that form the SEAM cell population.
[9] The production method according to the above [7], wherein the pluripotent stem cells are human iPS cells.

### ADVANTAGEOUS EFFECTS OF INVENTION

The present invention is able to produce corneal endothelial cells having characteristics more closely resembling those of corneal endothelial cells in a living body as compared with corneal endothelial cell-like cells produced by a conventional method. The present invention is able to provide corneal endothelial cells of sufficient quality to allow the cells to be transplanted into humans.

### BRIEF DESCRIPTION OF DRAWINGS

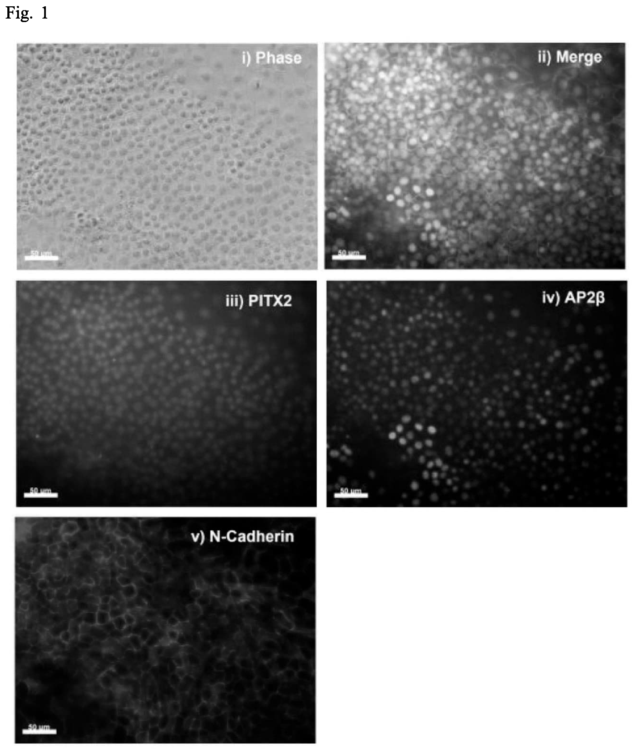

Fig. 1 shows the expression of human corneal endothelial cell markers (AP-2β, PITX2, and N-cadherin) observed by immunofluorescence staining in cells obtained as follows: a SEAM cell population induced from human iPS cells was detached and dispersed, and then cultured in a Matrigel-coated culture dish using a differentiation medium containing basic fibroblast growth factor (bFGF), and the resulting spheroids were cultured in a corneal endothelial induction medium containing bFGF.

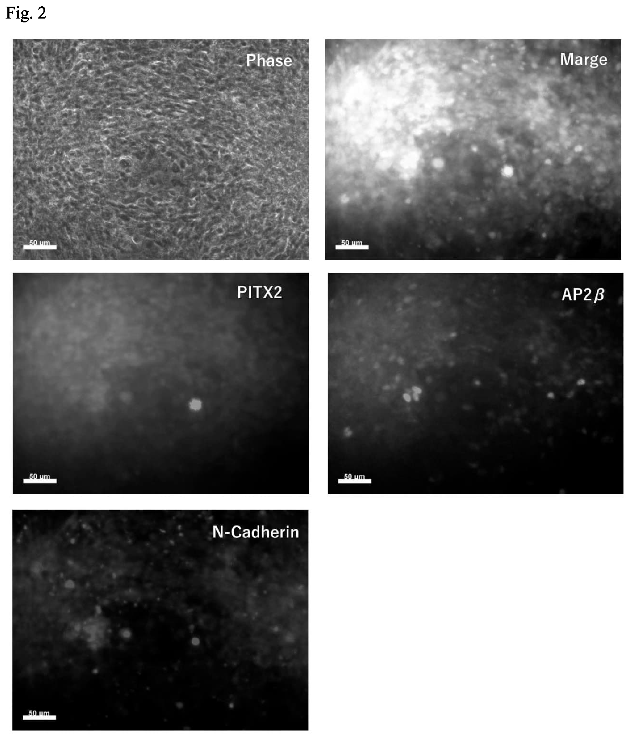

Fig. 2 shows the expression of human corneal endothelial cell markers (AP-2β, PITX2, and N-cadherin) observed by immunofluorescence staining in control cells for Fig. 1 obtained as follows: a SEAM cell population was detached and dispersed, and then cultured in a Matrigel-coated culture dish using a differentiation medium not containing bFGF, and the resulting spheroids were cultured in a corneal endothelial induction medium containing bFGF.

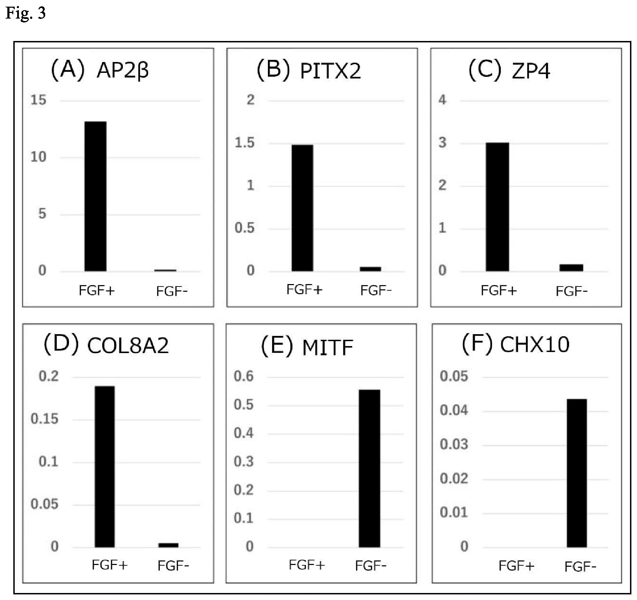

Fig. 3 shows the expression levels of periocular neural crest (POM) cell markers (AP-2β and PITX2), corneal endothelial cell markers (ZP4 and COL8A2), and neural retinal cell markers (MITF and CHX10) determined by quantitative RT-PCR in spheroids obtained as follows: a SEAM cell population induced from human iPS cells was detached and dispersed, and then cultured in a Matrigel-coated culture dish in a differentiation medium containing bFGF or a differentiation medium not containing bFGF.

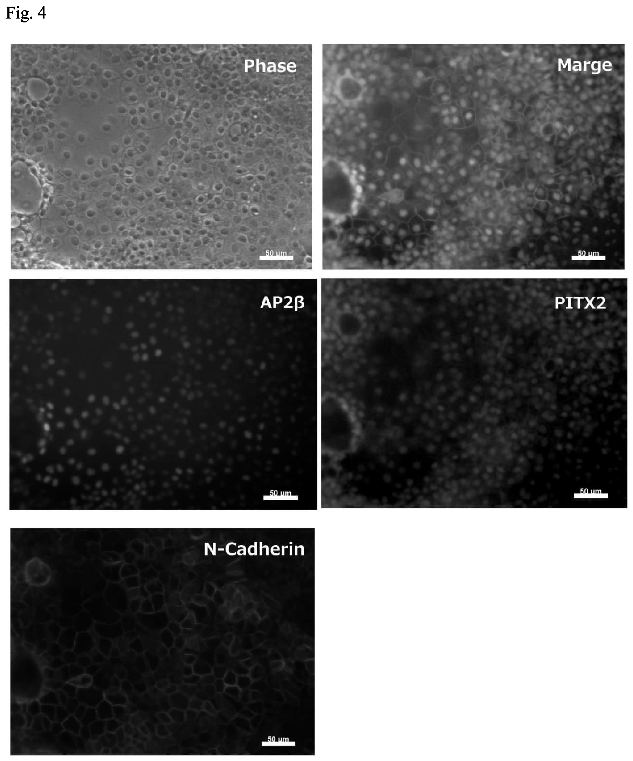

Fig. 4 shows the expression of human corneal endothelial cell markers (AP-2β, PITX2, and N-cadherin) observed by immunofluorescence staining in cells obtained as follows: a SEAM cell population induced from human iPS cells was detached and dispersed, and then cultured in a Matrigel-coated culture dish using a differentiation medium containing bFGF, and the resulting spheroids were cultured in a corneal endothelial induction medium containing bFGF.

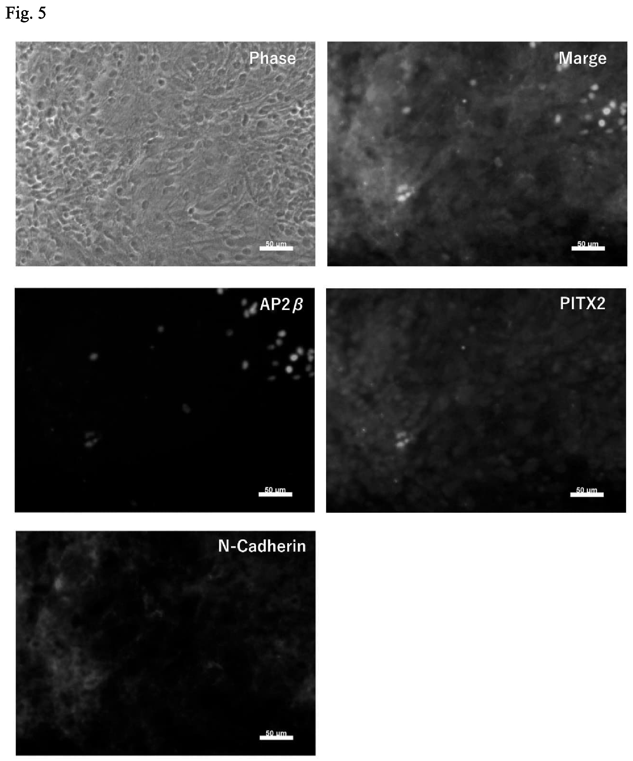

Fig. 5 shows the expression of human corneal endothelial cell markers (AP-2β, PITX2, and N-cadherin) observed by immunofluorescence staining in control cells for Fig. 4 obtained as follows: a SEAM cell population induced from human iPS cells was detached and dispersed, and then cultured in a Matrigel-coated culture dish using a differentiation medium containing bFGF, and the resulting spheroids were cultured in a corneal endothelial induction medium not containing bFGF.

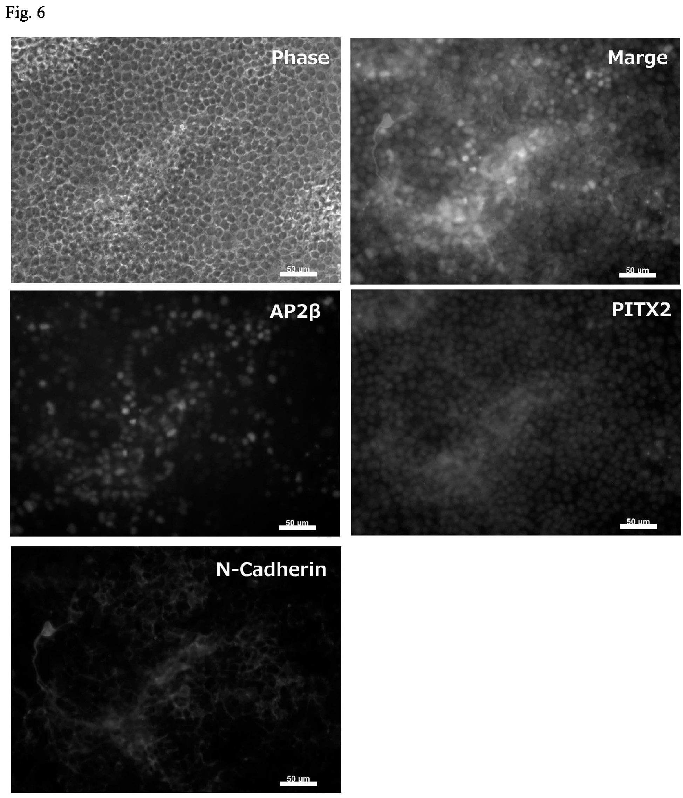

Fig. 6 shows the expression of human corneal endothelial cell markers (AP-2β, PITX2, and N-cadherin) observed by immunofluorescence staining in cells obtained as follows: a SEAM cell population induced from human iPS cells was detached and dispersed, and then cultured in a type IV collagen-coated culture dish using a differentiation medium containing bFGF, and the resulting spheroids were cultured in a corneal endothelial induction medium containing bFGF.

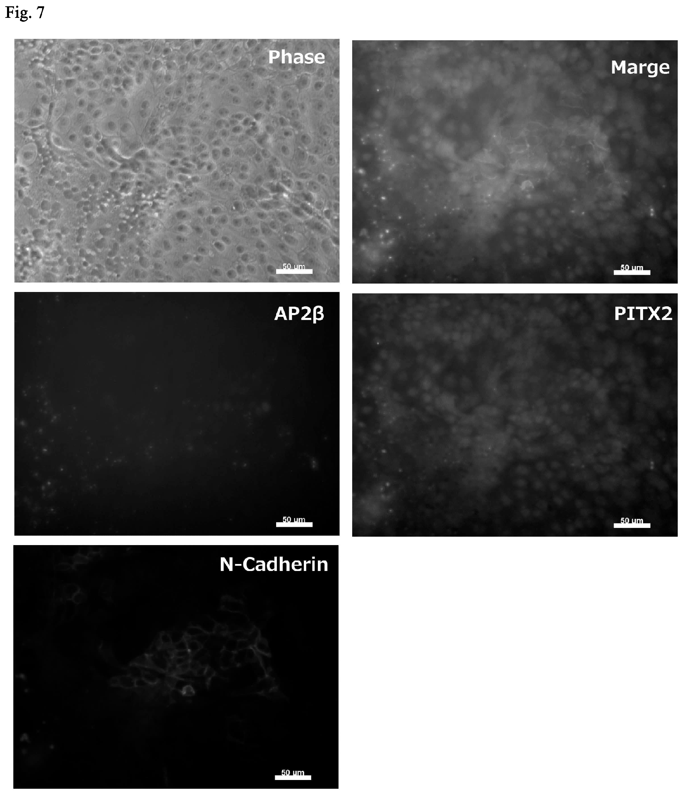

Fig. 7 shows the expression of human corneal endothelial cell markers (AP-2β, PITX2, and N-cadherin) observed by immunofluorescence staining in control cells for Fig. 6 obtained as follows: a SEAM cell population induced from human iPS cells was detached and dispersed, and then cultured in a type IV collagen-coated culture dish using a differentiation medium containing bFGF, and the resulting spheroids were cultured in a corneal endothelial induction medium not containing bFGF.

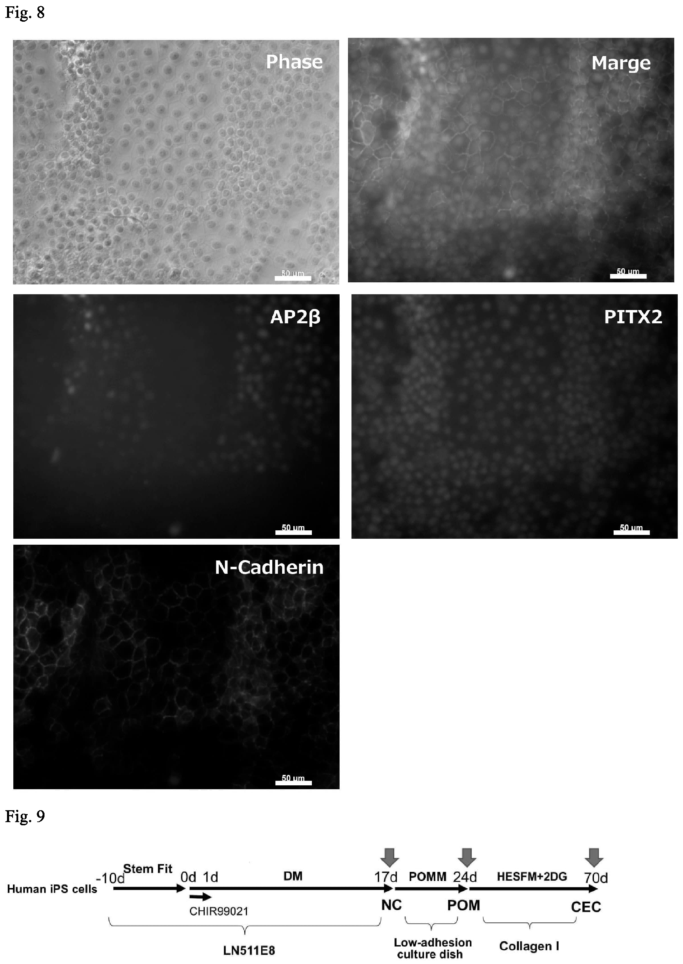

Fig. 8 shows the expression of human corneal endothelial cell markers (AP-2β, PITX2, and N-cadherin) observed by immunofluorescence staining in cells obtained as follows: a SEAM cell population induced from human iPS cells was detached and dispersed, and then cultured in a type IV collagen-coated culture dish using a differentiation medium containing bFGF, and the cells remaining adherent to the culture dish were cultured in a corneal endothelial induction medium containing bFGF.
Fig. 9 shows the conditions for the induction of differentiation of human iPS cells and reporter gene-introduced human iPS cells into corneal endothelial cells in Example 6.

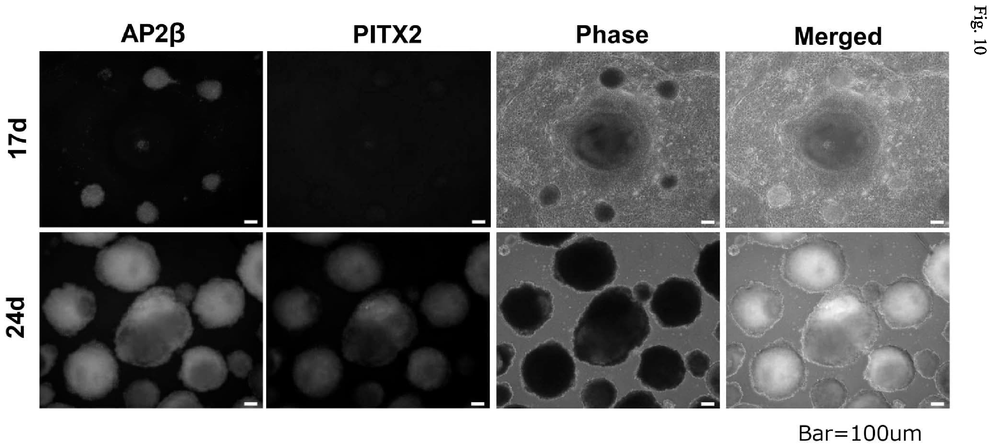

Fig. 10 shows the expression of a neural crest marker (AP-2β) and periocular neural crest (POM) cell markers (AP-2β and PITX2) examined via the expression of reporter genes (AP-2β is shown by green fluorescence and PITX2 is shown by red fluorescence) in spheroids at 17d recovered from a SEAM cell population induced from human iPS cells into which a reporter gene was introduced and in spheroids at 24d obtained by further culturing the spheroids at 17d in suspension culture in a low-adhesion culture dish using a periocular neural crest induction medium containing bFGF under the differentiation induction conditions shown in Fig. 9.

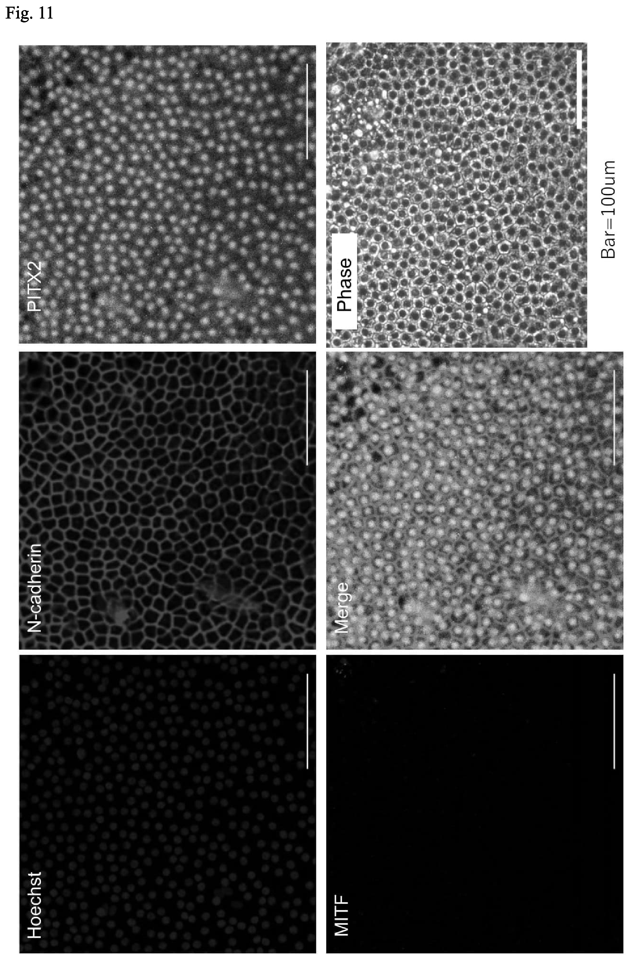

Fig. 11 shows the expression of human corneal endothelial cell markers (PITX2 and N-cadherin) and a retinal pigment epithelial cell marker (MITF) observed by immunofluorescence staining in cells at 70d induced by culturing human iPS cells under the differentiation induction conditions shown in Fig. 9.

### DESCRIPTION OF EMBODIMENTS

### Method for producing corneal endothelial cells

The present invention provides a method for producing corneal endothelial cells. The method for producing corneal endothelial cells of the present invention includes culturing periocular neural crest cells using a medium containing a fibroblast growth factor (FGF). The term "fibroblast growth factor" may be abbreviated to "FGF" as used herein. Periocular neural crest cells may also be referred to as periocular mesenchymal (POM) cells. The term "periocular neural crest" may be abbreviated to "POM" as used herein.

FGFs are a class of growth factors associated with various life phenomena, such as embryogenesis, angiogenesis, and wound healing. In humans, 22 types of FGFs (FGF1 to FGF14, and FGF16 to FGF23) have been identified. FGF1 is known as acidic FGF (aFGF), and FGF2 is known as basic FGF (bFGF). The FGF used in the method for producing corneal endothelial cells of the present invention may be any FGF that contributes to the induction of differentiation of periocular neural crest cells into corneal endothelial cells. The FGF used in the method for producing corneal endothelial cells of the present invention may be basic FGF. The term "basic FGF" may be abbreviated to "bFGF" as used herein.

Periocular neural crest cells can be identified by the expression of periocular neural crest cell markers, including, for example, PITX2, AP-2β, FOXC1, etc. Corneal endothelial cells can be identified by the expression of corneal endothelial cell markers, including, for example, PITX2, AP-2β, N-cadherin, etc., or by the distinct structure in which hexagonal cells are regularly arranged in a cobblestone-like pattern. In particular, the confirmation of the expression of AP-2β as a corneal endothelial cell marker indicates that the produced corneal endothelial cells have characteristics more closely resembling those of corneal endothelial cells in a living body (Non-Patent Literature 4) and are of sufficient quality to allow the cells to be transplanted into humans.

The medium containing a fibroblast growth factor (FGF) is not particularly limited, and may be a medium prepared by adding an FGF to a known medium that can be used for the culture of an animal cell. Examples of the known animal cell culture medium include, for example, BME medium, BMJb medium, CMRL 1066 medium, Glasgow MEM (GMEM) medium, Improved MEM Zinc Option medium, Neurobasal medium, IMDM medium, Medium 199, Eagle MEM medium, αMEM medium, DMEM medium, F-12 medium, DMEM/F12 medium, IMDM/F12 medium, Ham medium, RPMI 1640 medium, Fischer's medium, and a mixed medium thereof. The medium containing an FGF may be a medium prepared by adding an FGF to a known endothelial cell culture medium. The medium may be a serum-free medium. The serum-free medium may be suitably supplemented with a commercially available serum-free supplement, such as KnockOut Serum Replacement (KSR), N2 supplement, B27 supplement, etc. When corneal endothelial cells for transplantation are intended to be produced, a xeno-free medium is preferably used.

The concentration of an FGF in the medium is not particularly limited. For example, when bFGF is used, the concentration of bFGF in the medium may be 1 ng/mL or more, 2 ng/mL or more, 3 ng/mL or more, 4 ng/mL or more, or 5 ng/mL or more, or 10 ng/mL or less, 9 ng/mL or less, 8 ng/mL or less, 7 ng/mL or less, or 6 ng/mL or less. The concentration of bFGF in the medium may be 2 ng/mL or 4 ng/mL.

The medium containing an FGF used in the method for producing corneal endothelial cells of the present invention may further contain a glycolytic inhibitor. The glycolytic inhibitor may be any inhibitor that inhibits the glycolytic pathway to inhibit ATP production, and may be, for example, 2-deoxy-D-glucose (2DG). The concentration of 2DG in the medium is not particularly limited, and may be 2 mM or more, 4 mM or more, 6 mM or more, or 8 mM or more, or 20 mM or less, 18 mM or less, 16 mM or less, 14 mM or less, or 12 mM or less. The concentration of 2DG in the medium may be 10 mM.

The length of the culture period is not particularly limited and can be set as appropriate based on observations of corneal endothelial cell marker-expressing cells and/or the characteristic structure of corneal endothelial cells. The culture period may be, for example, 5 weeks or more, 6 weeks or more, 7 weeks or more, 8 weeks or more, 9 weeks or more, or 10 weeks or more.

The periocular neural crest cells used in the method for producing corneal endothelial cells of the present invention are not particularly limited, and may be periocular neural crest cells obtained by a known method. The known method for producing periocular neural crest cells may include, for example, the method described in Atkinson-Leadbeater et al. (Dev Dyn. 2014 May;243(5):663-75. doi: 10.1002/dvdy.24113. Epub 2014 Feb 24.), or the method described in Chen et al. (Invest Ophthalmol Vis Sci. 2018 Jun 1;59(7):3028-3036. doi: 10.1167/iovs.17-23627.).

The periocular neural crest cells used in the method for producing corneal endothelial cells of the present invention are preferably periocular neural crest cells induced to differentiate from pluripotent stem cells via a SEAM cell population. The method for inducing a SEAM cell population from pluripotent stem cells is known, and the method described in, for example, Non-Patent Literature 2 or 3 can be used. The known method for inducing periocular neural crest cells from pluripotent stem cells via a SEAM cell population may include, for example, the method described in Okubo et al. (J Biol Chem. 2020 Mar 13;295(11):3456-3465. doi: 10.1074/jbc.RA119.010713. Epub 2020 Feb 7.).

The pluripotent stem cells may include embryonic stem (ES) cells, embryonic stem cells from clone embryos obtained by nuclear transplantation (ntES cells), spermatogonial stem cells (GS cells), embryonic germ (EG) cells, induced pluripotent stem (iPS) cells, pluripotent cells (Muse cells) derived from cultured fibroblasts or myeloid stem cells, etc. Preferred are ES cells, ntES cells and iPS cells, and more preferred are iPS cells. The pluripotent stem cells are preferably pluripotent stem cells from a mammal. The mammal is not particularly limited, and examples thereof include humans, non-human primates, dogs, cats, mice, rats, cows, pigs, sheep, etc. Especially preferred are humans. When human pluripotent stem cells are used in the production method of the present invention, a cell population that can be safely used for human regenerative therapy can be obtained according to the cell type of interest.

The present inventors have reported that neural crest cells are contained in a SEAM cell population (Non-Patent Literature 2). Therefore, corneal endothelial cells produced by the method for producing corneal endothelial cells of the present invention using periocular neural crest cells induced to differentiate from pluripotent stem cells via a SEAM cell population are corneal endothelial cells that have differentiated through the developmental process of the human eye, have characteristics equivalent to those of corneal endothelial cells in a living body, and can be used for transplantation into humans.

### Method for producing periocular neural crest cells

The present invention also provides a method for producing periocular neural crest (POM) cells. The method for producing POM cells of the present invention includes forming a SEAM cell population from pluripotent stem cells, and culturing cells that form the SEAM cell population using a medium containing a fibroblast growth factor, wherein the cells contain neural crest cells. The POM cells produced by the method for producing POM cells of the present invention are suitable for use as POM cells for the method for producing corneal endothelial cells of the present invention described above. The POM cells produced by the method for producing POM cells of the present invention are also suitable for use in the production of various somatic cells arising from differentiation via POM cells.

The step of forming a SEAM cell population from pluripotent stem cells can be performed by a known method described in, for example, Non-Patent Literature 2 or 3.

### (1) First embodiment

A first embodiment of the method for producing POM cells of the present invention is a method including dispersing cells that form a SEAM cell population. In particular, the method can be performed by detaching a SEAM cell population, dispersing the cells that form the SEAM cell population, and culturing the dispersed cells in adherent culture using a medium containing an FGF. That is, in the first embodiment of the method for producing POM cells of the present invention, cells obtained by detaching and dispersing a SEAM cell population can be used as the "cells that form the SEAM cell population, wherein the cells contain neural crest cells" described above. As the present inventors have reported that neural crest cells are contained in a SEAM cell population (Non-Patent Literature 2), the confirmation of the presence of neural crest cells in the dispersed cells from a SEAM cell population may be omitted. The FGF is not particularly limited and may be bFGF. The neural crest cells in the dispersed cells from a SEAM cell population can be identified as cells expressing a neural crest cell marker. The neural crest cell marker may be, for example, SOX10, SOX9, AP-2β, p75NTR, etc.

The medium containing an FGF is not particularly limited, and may be a medium prepared by adding an FGF to a known medium that can be used for the culture of an animal cell, as with the medium used in the method for producing corneal endothelial cells of the present invention. The concentration of an FGF in the medium is not particularly limited. For example, when bFGF is used, the concentration of bFGF may be 1 ng/mL or more, 5 ng/mL or more, 7 ng/mL or more, 8 ng/mL or more, 9 ng/mL or more, or 10 ng/mL or more, or 20 ng/mL or less, 15 ng/mL or less, 13 ng/mL or less, 12 ng/mL or less, or 11 ng/mL or less. A suitable medium containing an FGF may be, for example, a medium prepared by adding an FGF to the differentiation medium as described later in Example 1.

A culture dish coated with an extracellular matrix may be used for the culture. The extracellular matrix may be any known extracellular matrix used for cell culture. Examples of the extracellular matrix include Matrigel (trade name), type I collagen, type IV collagen, laminin, fibronectin, vitronectin, etc.

In the first embodiment, when the dispersed cells from a SEAM cell population are cultured in adherent culture, the cells grow simultaneously into floating spheroid-forming cells and cells remaining adherent to the culture dish without forming spheroids, during the culture period. The present inventors have confirmed that POM cells reside in both the spheroid-forming cells and the cells remaining adherent to the culture dish. Therefore, the spheroid cells may be recovered to be used in the method for producing corneal endothelial cells of the present invention, or the cells remaining adherent to the culture dish may be used in the method for producing corneal endothelial cells of the present invention. Alternatively, the spheroid cells and the adherent cells may not be separated and may be used together in the method for producing corneal endothelial cells of the present invention.

The length of the culture period is not particularly limited and can be set as appropriate based on the confirmation of the emergence of cells expressing a POM cell marker. The culture period may be 5 days or more, 1 week or more, 2 weeks or more, 3 weeks or more, 4 weeks or more, 5 weeks or more, 6 weeks or more, or 7 weeks or more, or 15 weeks or less, 12 weeks or less, 10 weeks or less, 9 weeks or less, or 8 weeks or less.

### (2) Second embodiment

A second embodiment of the method for producing POM cells of the present invention can be performed by recovering spheroids formed from the second zone of a SEAM cell population, and culturing the spheroids in a medium containing an FGF. That is, in the second embodiment of the method for producing POM cells of the present invention, spheroids formed from the second zone of a SEAM cell population can be used as the "cells that form the SEAM cell population, wherein the cells contain neural crest cells" described above. As the present inventors have reported that after a SEAM cell population is cultured, neural crest cells are found in spheroids formed by the cells of the second zone (Non-Patent Literature 2), the confirmation of the presence of neural crest cells in the spheroids may be omitted. The FGF is not particularly limited and may be bFGF.

The medium containing an FGF may be a medium prepared by adding an FGF to a known medium that can be used for the culture of an animal cell, as with the medium used in the method for producing corneal endothelial cells of the present invention. The concentration of an FGF in the medium is not particularly limited. For example, when bFGF is used, the concentration of bFGF in the medium may be 1 ng/mL or more, 2 ng/mL or more, 3 ng/mL or more, 4 ng/mL or more, or 5 ng/mL or more, or 10 ng/mL or less, 9 ng/mL or less, 8 ng/mL or less, 7 ng/mL or less, or 6 ng/mL or less. Preferably, the medium containing an FGF used in the second embodiment further contains a ROCK inhibitor. A suitable medium containing an FGF may be, for example, the periocular neural crest induction medium (POMM) as described later in Example 6.

The culture dish is preferably a culture dish with low-adhesion surface treatment, but is not limited thereto. The length of the culture period is not particularly limited and can be set as appropriate based on the confirmation of the emergence of cells expressing a POM cell marker. The culture period may be 4 days or more, 5 days or more, 6 days or more, or 7 days or more, or 12 days or less, 11 days or less, 10 days or less, 9 days or less, or 8 days or less. The spheroids containing neural crest cells are small at the start of culture, but grow and differentiate during the culture period, and can be recovered as large spheroids containing POM cells.

### Method for producing corneal endothelial cells from pluripotent stem cells

The present invention provides a method for producing corneal endothelial cells from pluripotent stem cells, the method including the first embodiment of the method for producing POM cells of the present invention as described above, combined with the subsequent method for producing corneal endothelial cells of the present invention as described above. The present invention also provides a method for producing corneal endothelial cells from pluripotent stem cells, the method including the second embodiment of the method for producing POM cells of the present invention as described above, combined with the subsequent method for producing corneal endothelial cells of the present invention as described above. These methods of the present invention are able to provide corneal endothelial cells induced to differentiate through the developmental process of the human eye.

### EXAMPLES

The present invention will be described in more detail below with reference to Examples, but the present invention is not limited thereto.

### Example 1: Investigation of method for inducing differentiation of human iPS cells into human corneal endothelial cells 1

### (1) Induction of differentiation of iPS cells into SEAM cell population

The human iPS cell line 201B7 was obtained from Riken BioResource Research Center. The cells were seeded in a culture dish coated with laminin-511E8 (iMatrix-511 silk, Nippi, Inc.) at 0.5 µg/cm², and maintained in StemFit medium (Ajinomoto Co., Inc.) for 10 days. The cells were then cultured for 17 days in a differentiation medium (DM; GMEM (Life Technologies) supplemented with 10% knockout serum replacement (KSR, Life technologies), 1 mM sodium pyruvate (Life Technologies), 0.1 mM non-essential amino acids (Life Technologies), 2 mM L-glutamine (Life Technologies), 55 µM monothioglycerol (Wako), penicillin, and streptomycin) to induce a SEAM cell population. A SEAM cell population contains neural crest cells as previously reported by the present inventors (see Non-Patent Literature 2), and thus neural crest markers were not examined in Examples 1 to 5.

### (2) Culture of dispersed SEAM cell population

The obtained SEAM cell population was washed with PBS, and the cells were detached with Accutase. The detached cells were recovered by centrifugation and suspended in the differentiation medium (DM). The differentiation medium containing 10 ng/mL bFGF (FUJIFILM) (FGF+), or the differentiation medium not containing bFGF (FGF-) was used. The cells were then seeded in a 1% Matrigel-coated 12-well culture plate (containing 1 mL of DM) at 1.4 × 10⁶ cells/mL and cultured in adherent culture for 12 weeks. During the culture period, floating spherical cell aggregates (spheroids) were formed in the culture dish regardless of the presence or absence of bFGF.

### (3) Recovery of spheroids and induction of differentiation into human corneal endothelial cells by adherent culture

At 12 weeks of culture, the spheroids in the culture dish were recovered and cultured in adherent culture for 6 weeks in a 24-well culture plate (Falcon) using a corneal endothelial induction medium (HESFM; Human Endothelial SFM (Gibco) supplemented with 5% FBS, 0.3 mM L-ascorbic acid 2-phosphate, 1 µM SB431542, 10 µM Y-27632, penicillin, streptomycin, and 2 ng/mL bFGF).

### (4) Immunofluorescence staining

Immunofluorescence staining of the cells was performed after 6 weeks of adherent culture. The medium was removed, and the cells were fixed by adding 4% paraformaldehyde to each well. The cells were washed with Tris-buffered saline (TBS, TaKaRa Bio) three times for 10 minutes each. The cells were incubated with 5% donkey serum in TBS containing 0.3% Triton X-100 for 1 hour to block nonspecific reactions. The cells were then incubated with primary antibodies for the detection of human corneal endothelial cell markers at 4°C overnight. Then, the cells were washed with TBS three times for 10 minutes each. The cells were incubated with Alexa Fluor 488-, 568-, or 647-conjugated secondary antibodies (Life Technologies) at 1:500 dilution and Hoechst 33342 (Molecular Probes) at 1:100 dilution at 4°C overnight. The cells were then washed with TBS three times for 10 minutes each. The primary antibodies used were anti-AP-2β antibody (Cell Signaling Technology, #2509, Rabbit), anti-PITX2 antibody (abcam, ab55599, Mouse), and anti-N-cadherin antibody (R&D Systems, AF6426, Sheep). The cells were observed under a fluorescence microscope (Axio Observer.D1, Carl Zeiss).

### (5) Results

The images of immunofluorescence staining of the FGF+ group are shown in Fig. 1, and the images of immunofluorescence staining of the FGF- group are shown in Fig. 2. The FGF+ group exhibited a distinct structure in which hexagonal cells were regularly arranged in a cobblestone-like pattern, which is characteristic of human corneal endothelial cells (see the phase-contrast microscopic image), and expressed the human corneal endothelial cell markers, AP-2β, PITX2, and N-cadherin. On the contrary, the FGF- group showed no characteristic structure or expression of the human corneal endothelial cell markers.

### Example 2: Investigation of method for inducing differentiation of human iPS cells into human corneal endothelial cells 2

### (1) Induction of differentiation of iPS cells into SEAM cell population

A SEAM cell population was induced in the same manner as in the above (1) in Example 1, except that the culture period was changed to 21 days.

### (2) Culture of dispersed SEAM cell population

The SEAM cell population was detached, and cell suspensions were prepared using the differentiation medium containing bFGF (FGF+) or the differentiation medium not containing bFGF (FGF-) and seeded in a 1% Matrigel-coated culture dish, in the same manner as in the above (2) in Example 1. The cells were then cultured in adherent culture for 5 days, and spheroids in the culture dish were recovered.

### (3) Quantitative RT-PCR

Total RNA was extracted from the recovered spheroids using Sepasol-RNA I Super G (Nacalai Tesque, Cat. 09379-55). The total RNA was reverse-transcribed using SuperScript III First-Strand Synthesis System for RT-PCR (Thermo Fisher) following the manufacturer's protocol. The cDNA was used as a template for PCR. SYBR Green (Thermo Fisher) was used as a fluorescent probe. The thermal cycling program includes the initial cycle of 95°C for 20 seconds, followed by 40 cycles of 95°C for 3 seconds, and then 95°C for 15 seconds and 60°C for 60 seconds. The following primers were used:
AP2β-F: TTCCTCCCAAATCGGTGACTT (SEQ ID NO: 1),
AP2β-R: CGCCGGTGTTGACAGACAT (SEQ ID NO: 2),
PITX2-F: GCCAAGGGCCTTACATCCG (SEQ ID NO: 3),
PITX2-R: GGTGGGGAAAACATGCTCTG (SEQ ID NO: 4),
ZP4-F: GACTGTGGCACCTGGATAAGA (SEQ ID NO: 5),
ZP4-R: TCCCACTCAGTGACATAGCAG (SEQ ID NO: 6),
COL8A2-F: GAGCCAGGAATACGAGGGGA (SEQ ID NO: 7),
COL8A2-R: TCCAGGGATAGTAATGCCTGAG (SEQ ID NO: 8),
MITF-F: AGAGTCTGAAGCAAGAGCACTG (SEQ ID NO: 9),
MITF-R: TGCGGTCATTTATGTTAAATCTTC (SEQ ID NO: 10),
CHX10-F: GGCGACACAGGACAATCTTTA (SEQ ID NO: 11), and
CHX10-R: GGCAGCTCCGTTTTCATGG (SEQ ID NO: 12).

### (4) Results

The results are shown in Fig. 3. The panel (A) shows AP-2β (POM marker), the panel (B) shows PITX2 (POM marker), the panel (C) shows ZP4 (corneal endothelial cell marker), the panel (D) shows COL8A2 (corneal endothelial cell marker), the panel (E) shows MITF (neural retinal cell marker), and the panel (F) shows CHX10 (neural retinal cell marker). The results demonstrate that the cells of the FGF+ group express the POM markers AP-2β and PITX2 and the corneal endothelial cell markers ZP4 and COL8A2, but do not express the neural retinal cell markers MITF and CHX10. In contrast to the FGF+ group, the cells of the FGF- group express the neural retinal cell markers (MITF and CHX10), but show almost no expression of the POM markers (AP-2β and PITX2) and the corneal endothelial cell markers (ZP4 and COL8A2). The results reveal that FGF is essential for the induction of differentiation of neural crest cells contained in the SEAM cell population into POM.

### Example 3: Investigation of usefulness of FGF in induction of differentiation of spheroids formed in medium containing FGF into corneal endothelial cells 1

### (1) Recovery and culture of spheroids

A SEAM cell population was induced in the same manner as in the above (1) in Example 1, except that the culture period was changed to 14 days. The SEAM cell population was detached, and a cell suspension was prepared using the differentiation medium containing bFGF, seeded in a 1% Matrigel-coated 12-well culture plate, and cultured for 12 weeks, in the same manner as in the above (2) in Example 1. At 12 weeks of culture, the spheroids in the culture dish were recovered and cultured in adherent culture for 9 weeks in a 24-well culture plate using the corneal endothelial induction medium containing bFGF (FGF+) or the corneal endothelial induction medium not containing bFGF (FGF-).

### (2) Immunofluorescence staining

Immunofluorescence staining of the cells was performed after 9 weeks of adherent culture in the same manner as in the above (4) in Example 1.

### (3) Results

The images of immunofluorescence staining of the FGF+ group are shown in Fig. 4, and the images of immunofluorescence staining of the FGF- group are shown in Fig. 5. The FGF+ group exhibited a distinct structure in which hexagonal cells were regularly arranged in a cobblestone-like pattern, which is characteristic of human corneal endothelial cells (see the phase-contrast microscopic image), and expressed the human corneal endothelial cell markers, AP-2β, PITX2, and N-cadherin. On the contrary, the FGF- group showed only a small number of cells with the characteristic structure of human corneal endothelial cells, and had lower expression levels of the human corneal endothelial cell markers. The results reveal that FGF is essential for the induction of differentiation of POM into corneal endothelial cells.

### Example 4: Investigation of usefulness of FGF in induction of differentiation of spheroids formed in medium containing FGF into corneal endothelial cells 2

### (1) Experimental method

The experiment was performed in the same manner as in Example 3, except that the extracellular matrix for coating the culture plate was changed from Matrigel to type IV collagen (Cellmatrix Type IV, Nitta Gelatin Inc.). Cellmatrix Type IV (3 mg/mL) was diluted to 20-fold in hydrochloric acid (pH 3) and used to coat the culture dish.

### (2) Results

The images of immunofluorescence staining of the FGF+ group are shown in Fig. 6, and the images of immunofluorescence staining of the FGF- group are shown in Fig. 7. The FGF+ group exhibited a distinct structure in which hexagonal cells were regularly arranged in a cobblestone-like pattern, which is characteristic of human corneal endothelial cells (see the phase-contrast microscopic image), and expressed the human corneal endothelial cell markers, AP-2β, PITX2, and N-cadherin. On the contrary, the FGF- group showed only a small number of cells with the characteristic structure of human corneal endothelial cells, and had lower expression levels of the human corneal endothelial cell markers. The results reveal that the same results as those of the preceding experiment are obtained even when the extracellular matrix for coating the culture dish is changed to a different type.

### Example 5: Induction of differentiation of residual adherent cells remaining after recovery of spheroids formed in medium containing FGF into corneal endothelial cells

### (1) Experimental method

A dispersed SEAM cell population was seeded in a type IV collagen-coated 12-well culture plate and cultured for 12 weeks, and spheroids in the culture dish were recovered, in the same manner as in Example 4. Then, adherent cells remaining on the culture dish were cultured for 9 weeks in the corneal endothelial induction medium containing bFGF. After 9 weeks of adherent culture, immunofluorescence staining of the cells was performed in the same manner as in the above (4) in Example 1.

### (2) Results

The results are shown in Fig. 8. The cells exhibited a distinct structure in which hexagonal cells were regularly arranged in a cobblestone-like pattern, which is characteristic of human corneal endothelial cells (see the phase-contrast microscopic image), and expressed the human corneal endothelial cell markers, AP-2β, PITX2, and N-cadherin. The results reveal that the periocular neural crest (POM) cells induced to differentiate from the dispersed SEAM cell population by culturing the dispersed SEAM cell population in the differentiation medium containing FGF are present not only in the cells that form spheroids but also in the adherent cells that do not form spheroids.

### Example 6: Investigation of usefulness of 2DG in induction of differentiation of POM into corneal endothelial cells

### (1) Differentiation induction conditions

The conditions for the induction of differentiation of human iPS cells into corneal endothelial cells in this Example are shown in Fig. 9. The human iPS cells used in this Example were the 201B7 cell line and an AP2β-EGFP/PITX2-E2-Crimson reporter iPS cell line. The reporter iPS cell line was prepared by transfection of iPS cells with a targeting gene containing a drug resistance gene via genome editing using CRISPR/Cas9. The human iPS cells were maintained in StemFit medium for 10 days (0d), and then cultured for 1 day in the differentiation medium (DM) supplemented with 3 µM CHIR99021, in the same manner as in Example 1. The cells were cultured for 16 days in the differentiation medium not supplemented with CHIR99021 to induce a SEAM cell population. The clusters of neural crest cells generated in zone 2 of the SEAM cell population were recovered as spheroids (17d). The recovered spheroids were transferred to a low-adhesion culture dish (Sumitomo Bakelite Co., Ltd., MS-1160R), and the spheroids were cultured in suspension culture for 7 days in a periocular neural crest induction medium (POMM; DMEM/F12 supplemented with 20% knockout serum replacement, 1 mM sodium pyruvate, 0.1 mM non-essential amino acids, 2 mM L-glutamine, 55 µM monothioglycerol, 10 µM Y-27632, penicillin, streptomycin, and 4 ng/mL bFGF) to induce POM. The spheroid-shaped POM was recovered (24d). The recovered spheroid-shaped POM was seeded in a type I collagen (Cellmatrix Type I-A, Nitta Gelatin Inc.)-coated culture dish, and cultured until day 70 (70d) in the corneal endothelial induction medium described in the above (3) in Example 1 supplemented with 10 mM 2-deoxy-D-glucose (2DG).

### (2) Immunofluorescence staining at 17d and 24d

Expression of AP-2β in the cells at 17d and 24d was examined via the expression of the AP2β-EGFP reporter gene (green fluorescence). Expression of PITX2 in the cells at 17d and 24d was examined via the expression of the PITX2-E2-Crimson reporter gene (red fluorescence).

### (3) Immunofluorescence staining at 70d

Immunofluorescence staining of the cells at 70d was performed in the same manner as in the above (4) in Example 1. The primary antibodies used were an anti-PITX2 antibody, an anti-N-cadherin antibody, and an anti-MITF antibody. PITX2 and N-cadherin are corneal endothelial cell markers. MITF is a retinal pigment epithelial cell marker and is used as a negative control.

### (4) Results

The expression of the reporter genes at 17d and 24d is shown in Fig. 10. The clusters (spheroids) of AP-2β-positive neural crest cells were observed in the SEAM cell population induced by culture in the DM medium for 17 days. The spheroids at 24d obtained by culturing the spheroids of the neural crest cells recovered at 17d in the periocular neural crest induction medium are positive for AP-2β and PITX2, indicating that the spheroids differentiated into POM.

The images of immunofluorescence staining at 70d are shown in Fig. 11. When the spheroids of POM recovered at 24d were cultured in adherent culture in a type I collagen-coated culture dish using the corneal endothelial induction medium (HESFM) containing bFGF and 2DG, the resulting spheroids exhibited a distinct structure in which hexagonal cells were regularly arranged in a cobblestone-like pattern, which is characteristic of human corneal endothelial cells. The spheroids expressed the human corneal endothelial cell markers PITX2 and N-cadherin, but lacked the expression of the retinal pigment epithelial cell marker MITF.

The present invention is not limited to each of the embodiments or Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

## Claims

1. A method for producing corneal endothelial cells, comprising culturing periocular neural crest cells using a medium containing a fibroblast growth factor.

2. The production method according to claim 1, wherein the medium further contains a glycolytic inhibitor.

3. The production method according to claim 2, wherein the glycolytic inhibitor is 2-deoxy-D-glucose.

4. The production method according to claim 1, wherein the periocular neural crest cells are periocular neural crest cells induced to differentiate from pluripotent stem cells.

5. The production method according to claim 1, wherein the periocular neural crest cells are periocular neural crest cells induced to differentiate from pluripotent stem cells via a SEAM cell population.

6. The production method according to claim 4 or 5, wherein the pluripotent stem cells are human iPS cells.

7. A method for producing periocular neural crest cells, comprising forming a SEAM cell population from pluripotent stem cells, and culturing cells that form the SEAM cell population using a medium containing a fibroblast growth factor, wherein the cells contain neural crest cells.

8. The production method according to claim 7, wherein the culturing cells that form the SEAM cell population using a medium containing a fibroblast growth factor comprises dispersing the cells that form the SEAM cell population.

9. The production method according to claim 7, wherein the pluripotent stem cells are human iPS cells.
